# EUROPEAN PATENT SPECIFICATION

(11) **EP 2 909 303 B1**
(45) Date of publication and mention of the grant of the patent: **15.04.2020**
(21) Application number: 13876387.5
(22) Date of filing: 29.09.2013
(51) Int. Cl.: C12M 3/00, C12M 1/06, C12M 1/36

(54) **BIOREACTOR SYSTEM AND METHODS FOR ALTERNATIVE CELL CULTURE BETWEEN STATIC AND DYNAMIC**
BIOREAKTORSYSTEM UND VERFAHREN FÜR ALTERNATIVE ZELLKULTUREN ZWISCHEN STATIK UND DYNAMIK
SYSTÈME DE BIORÉACTEUR ET PROCÉDÉS DE CULTURE CELLULAIRE ALTERNANT ENTRE UN ÉTAT STATIQUE ET UN ÉTAT DYNAMIQUE

(43) Date of publication of application: 26.08.2015
(73) Proprietor: Zhang, Yongxin, Carrollton, TX 75007 (US)
(72) Inventor: Zhang, Yongxin, Carrollton, TX 75007 (US)
(74) Representative: TBK
(86) International application number: PCT/IB2013/003260
(87) International publication number: WO 2014/132101

(56) References cited:
- WO-A2-2005/003332
- KR-A- 20080 077 542
- US-A1- 2005 054 101
- US-A1- 2005 054 101
- US-A1- 2006 019 388
- US-A1- 2007 026 516
- US-A1- 2012 164 680
- BARZEGARI, ABOLFAZL ET AL.: 'An update to space biomedical research: tissue engineering in microgravity bioreactors' BIOIMPACTS vol. 2, no. 1, 16 March 2012, pages 23 - 32, XP055254027
- MARTIN, IVAN ET AL.: 'The role of bioreactors in tissue engineering' TRENDS IN BIOTECHNOLOGY vol. 22, no. 2, 01 February 2004, pages 80 - 86, XP004486416

## Description

This application claims priority to United States Provisional Application No. 61/715555 which was filed October 18, 2012.

**Cross-References to Related application:**

| US Patents: | | |
|---|---|---|
| 5968820 | October 1999 | Zborowski |
| 6120735 | September 2000 | Zborowski |
| 7339045 | January 2009 | Oakey |
| 893569 | March 2005 | Felder |
| 7339045 | March 2008 | Rothschild |
| 8071395 | December 2011 | Davis |
| 8123199 | February 2012 | Terentiev |
| 5779359 | July 1998 | Gambrill et al. |
| 5803137 | September 1998 | Shimotoyodome et al |
| 5939313 | August 1999 | Cheng |
| 5941635 | August 1999 | Stewart |
| 5988422 | November 1999 | Vallot |
| 6071005 | June 2000 | Ekambaram et al. |
| 6245555 | June 2001 | Curtis |
| 6247840 | June 2001 | Gaffar |
| 6357907 | March 2002 | Cleveland et al. |
| 6379625 | April 2002 | Zuk, Jr. |
| 6402367 | June 2002 | Lu et al. |
| 6439756 | August 2002 | Forschner et al. |
| 6467946 | October 2002 | Gebrian |
| 6500343 | December 2002 | Siddiqi |
| 6514137 | February 2003 | Panelli et al. |
| 6555011 | April 2003 | Tribelsky et al. |
| 6637927 | October 2003 | Lu et al. |
| 6670171 | December 2003 | Carll |
| 6736906 | May 2004 | Cotte et al. |
| 6764859 | July 2004 | Kreuwel et al. |
| 6923567 | August 2005 | Bibbo et al. |
| 7153021 | December 2006 | Goodwin et al. |
| 7278780 | October 2007 | Goodwin et al. |
| 7384027 | June 2008 | Terentiev et al. |
| 7384783 | June 2008 | Kunas et al. |
| 7629167 | December 2009 | Hodge et al. |
| 7762716 | July 2010 | Terentiev et al. |
| 2001/0039369 | November 2001 | Terentiev |
| 2002/0082173 | June 2002 | Terentiev |
| 2002/0091371 | July 2002 | Ritter |
| 2002/0105856 | August 2002 | Terentiev |
| 2002/0145940 | October 2002 | Terentiev |
| 2003/0008389 | January 2003 | Carll |
| 2003/0170810 | September 2003 | Vedadi et al. |
| 2003/0226857 | December 2003 | Bibbo et al. |
| 2004/0047232 | March 2004 | Terentiev |
| 2004/0062140 | April 2004 | Cadogan et al. |
| 2004/0218468 | November 2004 | Terentiev |
| 2004/0221897 | November 2004 | Schubmehl et al |
| 2004/0229335 | November 2004 | Zhang et al. |
| 2004/0252582 | December 2004 | Bucher |
| 2005/0002274 | January 2005 | Terentiev |
| 2005/0117449 | June 2005 | Terentiev |
| 2005/0127215 | June 2005 | Lienhart et al. |
| 2005/0163667 | July 2005 | Krause |
| 2005/0201201 | September 2005 | Terentiev |
| 2005/0239199 | October 2005 | Kunas et al. |
| 2005/0272146 | December 2005 | Hodge et al. |
| 2006/0092761 | May 2006 | Terentiev |
| 2006/0131765 | June 2006 | Terentiev et al. |
| 2006/0270036 | November 2006 | Goodwin et al. |
| 2007/0030759 | February 2007 | Terentiev |
| 2007/0201993 | August 2007 | Terentiev et al. |
| 2007/0220956 | September 2007 | Terentiev |
| 2007/0252290 | November 2007 | Terentiev et al. |
| 2007/0263484 | November 2007 | Terentiev |
| 2008/0008028 | January 2008 | Terentiev et al. |

**Foreign Patent Documents**

| | | |
|---|---|---|
| 2271583 | Apr., 2002 | CA |
| 2017472 | Nov., 1971 | DE |
| 3246330 | Jun., 1984 | DE |
| 3407370 | Aug., 1985 | DE |
| 3818776 | Jul., 1989 | DE |
| 19542227 | May., 1997 | DE |
| 29800818 | Mar., 1998 | DE |
| 19705118 | Aug., 1998 | DE |
| 19917398 | Oct., 2000 | DE |
| 20114076 | Oct., 2001 | DE |
| 0033292 | Aug., 1981 | EP |
| 0200792 | Jan., 1985 | EP |
| 0200792 | Nov., 1986 | EP |
| 0343885 | Nov., 1989 | EP |
| 0433463 | Jun., 1991 | EP |
| 0590473 | Apr., 1994 | EP |
| 1462155 | Sep., 2004 | EP |
| 2799138 | May., 1999 | FR |
| 2076677 | Dec., 1981 | GB |
| 2202549 | Sep., 1988 | GB |
| 61-067476 | Apr., 1986 | JP |
| 61212275 | Sep., 1986 | JP |
| 631626 | Jan., 1988 | JP |
| 63-36825 | Feb., 1988 | JP |
| 03-242297 | Oct., 1991 | JP |
| 6153902 | Jun., 1994 | JP |
| 9-141079 | Dec., 1998 | JP |
| 10313718 | Dec., 1998 | JP |
| 10314569 | Dec., 1998 | JP |
| WO9833538 | Aug., 1998 | WO |
| 9852629 | Nov., 1998 | WO |
| WO0011953 | Mar., 2000 | WO |
| 03028869 | Apr., 2003 | WO |
| WO 2005/037658 | Apr., 2005 | WO |
| WO 2005/118771 | Dec., 2005 | WO |
| WO 2006/002091 | Jan., 2006 | WO |
| WO 2006/063087 | Jun., 2006 | WO |
| WO 2007/039600 | Apr., 2007 | WO |
| WO 2008/040567 | Apr., 2008 | WO |
| WO 2008/040568 | Apr., 2008 | WO |

### RELATED APPLICATIONS

This application is a continuation in part of PCT application with a Application Number PCT/US2012/000182, filed March 29, 2012 with a priority date March 29, 2011 from a provisional application with an Application Number 61/468573, filed March 29, 2011, and a continuation in part of U.S. application with an Application Number 13435250, filed on March 30, 2012. This application is also a continuation of U.S. provisional application wtih an Application Number 61/715555, filed on October 18, 2012.

### Field of the invention

The invention relates to a bioreactor for cultivating cells in three possible states: static, dynamic, or alternating between static and dynamic states as defined in claim 1.

### Background of the invention

This application relates generally to bioreactors and more particularly bioreactors for growing cells in three possible states: static, dynamic, or alternating between static and dynamic states as defined in claim 1.

Many kinds of cells, especially hematopoietic stem celts and immunocytes, are very sensitive to shear-stress In the culture. For example, shear-stress can cause the non-specific differentiation and the increased apoptosis in the stem cell culture, which significantly reduces the efficiency of the stem cell expansion and directed differentiation. The higher shear-stress also causes the more release of non-specific proteins in protein expression, in which the protein of interest takes less proportion in the culture and so result in the increased difficulties of protein purification. The static culture has the least shear stress but the cells in static culture normally sit at the bottom of the culture containers, some cells cannot get enough nutrition when cells are at higher density and so not suitable for large stale cell expansion. Some bioreactors, such as NASA's rotation wall vessel (RWV) bioreactor, were designed for reducing the shear-stress. However, these bioreactors have to keep cells in suspension by continuously moving, stirring or/and agitating cells. Once the bioreactor stop running, cells will accumulate or aggregate somewhere of the bottom of or other locations within the cell culture container but are not evenly distributed, which is harmful for most cell growth or at least is not conducive to efficient cell growth. Therefore, though the shear-stress has been reduced in these bioreactors, such a reduced shear-stress has to continuously exert on the cultured cells when these bioreactors are running. No doubt, an intermittent cell agitation, or the suitable alternation between static and dynamic culture, will not only minimize the sheer-stress but also provide cells an ideal metabolic environment. However, it is often very difficult to get cells evenly distributed in the static cell culture following a dynamic culture because of the inertia the moving cells have, while an uneven distribution (such as local accumulation or aggregation) of cells in static culture could be very harmful for cell growth as mentioned above.

Some other bioreactors, such as those invented by Felder and colleagues, the static and dynamic states of the cell cultures are respectively performed in two or more different cell culture containers and the changes between the two states of the cell culture are carried out by transferring cell from one type of cell culture container to the other type of cell culture container. When cells are transferred between containers, the cell losses and damages are inevitable. In our current invention, both static and dynamic cell cultures are carried out in the same cell culture container(s), no cell-transferring between cell culture container is needed during the alternating between the two culture states, and when the cell culture is changed from the dynamic to the static status, cells can be evenly distributed at the bottom of the culture chamber (container) or on the surface of the stirring or supporting materials. Thus, our invention provides cells the best growth condition in both suspension states and static states, and these two states can repeatedly alternate.

Some bioreactors use magnet element (specifically blades or vans) controlled by magnet impeller to agitate culture media to keep cells in suspension status. This kind of bioreactor purposely enhances the shear-stress for the culture requirements of a certain cells and the cells are distributed following the direction of media flow agitated by the impellers when the agitation stops. The higher shear-stress and uneven distribution of the cells in static state following agitation make it significant different from our current invented bioreactor system. In addition to the differences in the application, the bioreactor in our invention does not use blades or canes to be the magnet element, and the magnet beads in our invention actually has no magnetism if they are not in magnet field and they can only gain magnetism when they are placed in magnet field. The magnet beads in our invention are not controlled by impeller but by the changes of magnetic field strength affecting the beads' moving.

In one embodiment of our bioreactor, in order to minimize the sheer-stress during the agitation and allow the cells evenly distributed in the bioreactor, the agitators' movement between two ends of the interior cell culture container in combination with the corresponsive inversion of the cell culture container are applied in our current invented system. In comparison, other bioreactors either use agitators or use moving container to keep the cells suspended, and either of them does not allow the cells evenly distributed in the bioreactor following agitation and always exerts higher sheer-stress on cells in the culture. It also needs to be emphasized that the inversion of the cell culture container in our system is not for the cell suspension. Instead, the effects of the movement of the cell culture container on the cells suspension in this embodiment are intended to be minimized in our design, because the movement of the container often tends to result in uneven distribution of the cells in static culture. The inversion of cell culture container in our system mainly provides a suitable condition for the agitators to move from one end to the other end of the interior cell culture container.

In another embodiment of our bioreactor, the changes of cell culture states between static and dynamic are carried out by the changes between vertical rotating culture and horizontal static culture. The speed of the cell culture container rotation and the deceleration of the of the cell culture container from rotating to static state are two critical factors affecting cell distribution in static state. The higher or lower speed and deceleration of the cell culture container rotation can result in uneven distribution of the cells following rotation. In our research, the suitable speed and deceleration were founded.

In our research, we also found that both the time length of the static culture and dynamic culture and the frequency of the alternation between static culture and dynamic culture significantly affect the cell growth and need to be determined based upon the cell type, media, cell density (cell concentration) and cell doubling time (cell growth speed) for the specific cell culture.

In our previous patent application, some of above embodiments have been mentioned but not clearly shown in the claims. In this application, they are all clearly claimed.

### Summary

The invention relates to a bioreactor system as defined in claim 1.

In the invented bioreactor system, the combined application of the magnetically controlled agitation and the cell culture container inversion as well as the combined application of the vertical rotating culture and horizontal static culture are the two strategies in building the ideal bioreactors for the cell culture alternating between static and dynamic states in the same cell culture container, which can minimize the sheer-stress and provide cells an ideal metabolic environment.

Although the currently invented bioreactor system allows growth in all three culture states, especially in the alternation between static and dynamic, it can also used for either static cell culture or dynamic cell culture.

The even distribution of the cells in static culture said in the claim 2 can be on the bottom of the cell culture container or on any materials on the bottom of the cell culture container, or any part of the interior cell culture container.

The cells cultivated by the currently invented bioreactor system include all types of suspension cells, adherent cells and partial adherent cells. When the adherent cells are cultured, they can be attached to a certain carriers.

The dynamic state of the bioreactor system of claim 1 includes conditions in which cells move within the medium, the medium moves around the cells, the culture container moves or any combination thereof. When the cells move in the media, adherent cells moves with the agitators or carriers on which these cells attach. For the smaller sheer-stress during the cell re-suspension or agitation, the applications of vertical movement of agitators and vertical rotation of cell culture containers are two of the options, and other methods may also be used in this bioreactor system for the intermittent cell suspension culture.

The static state of this bioreactor system includes any conditions in which cells do not move relative to the cell culture container, cell culture medium, the bioreactor or the environment in which the cell culture is being performed. In some cases, the cells attached to some carriers and these carriers may be located at the side walls of the container and do not move relative to the cell culture container, it could be considered as static culture though these cells are not at the bottom as in usual static culture.

The cell culture states in this bioreactor system can freely alternate between the dynamic state of claim 4 and the static state of claim 5, in which the alternations can occur at any time, speed, and frequency, which may be operated by the program. We have found the relationships among the period of static or dynamic culture, the frequency of the static and dynamic culture alternation, cell density, cell growth speed (doubling time), cell yield and the cell quality which was evaluated by the cell death and apoptosis and cell non-specific differentiation and engraftment potential for stem cells.

The current invented bioreactor system may comprise a combination of a cell culture container inversion and magnet agitation. In one embodiment, cells are re-suspended by some agitators which were controlled by electric magnet field. When agitators are moving up, cells are brought up by these agitators. When the agitators reach the top of the cell culture container, they are held by the electric magnet at that end. Then cell container will be inverted about 180° to position the agitators at the lower part (bottom) of the container following the inversion. These agitations of cells in this case may start before the inversion of the cell culture container. The cell container inversion mainly provides suitable position for the agitators to move up and down.

The current invented bioreactor system may also comprise a combination of cell culture container orientation for rotating culture and another culture container orientation mainly for static culture. In one embodiment, the cell container rotates vertically and keeps its static culture horizontally. For the even distribution of cells in the static culture, the speed of the rotation, the deceleration of the cell culture container rotation and the speed of its position change from vertical to horizontal as well as their relationships have their optimal ranges. The range for the optimal speed of the rotation is 2 rpm to 60 rpm, the range for the deceleration of the cell culture container is -5rpm² to -120 rpm², the range for the ratio of the rotation speed /deceleration of the celt culture container rotation is 1 to 160, and the range for the speed of container position change is 0.1 to 60 rpm. In these ranges, cells can be or have significantly higher chance to be evenly distributed in the cell culture container in the static culture following a dynamic culture. In contrast, cells have significantly less chance to be or completely cannot be evenly distributed if the bioreactor setting is out of these ranges.

Although most bioreactors are controlled by computing system, since our current disclosed bioreactor system is capable to cultivate cells in all three states (static, dynamic, or alternating between static and dynamic states the static culture) in the same cell culture container(s), especially it can keep cells evenly distributed in static culture following a dynamic culture, the computing system controlling on this system for the three states adjustment is unique and important. The said computing control system includes all kinds of computers and similar automation devices which can be used to program the three states, especially used to program the alternation between static and dynamic states.

Cell culture container (vessel, chamber or sealed plate) in this invention can be made with any materials that are not harmful for the cell growth. It is preferable that at least one part of the cell culture container wall is made of the gas permeable material or dialysis membrane. The container has one or more ports serving as entrances or/and exits for cells, media, buffer, agitators and other materials, such as protein, cytokines, and other reagents, for cell growth and differentiation. The agitator can be in any shape, although a sphere in a suitable size is favorable. It could but not limited to be made of paramagnet (magnetizable) materials covered by some materials that are inert and good for cell growth and in some cases for cell attachment.

The bioreactor system has its power sets, which include but not limited to the engines, step motors, servo motors and so on. These power sets are used for most mechanical movement of any part of the bioreactor, including but not limited to the inversion and rotation of cell culture container, the adjustment of the vertical and horizontal level for the cell culture states.

### Brief Description of the Drawings

The drawings, when considered in connection with the following description, are presented for the purpose of facilitating an understanding of the subject matter sought to be protected.
FIG. 1 is a schematic view showing an illustrative bioreactor;
FIGS. 2a-2f show a series of schematic views of a bioreactor showing the movement of an agitator within a chamber in series;
FIGS. 3a-3b show a series of schematic views of a bioreactor with a cell culture container in the vertical rotation state (3a) and in the horizontal static state (3b).

### Detailed Description

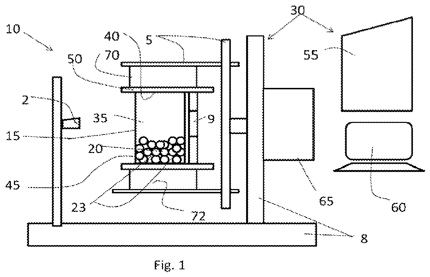

Referring now to FIG. 1, a bioreactor system 10 for growing cells is shown. The system 10 includes a cell culture chamber 15, an agitator 20 and a control system 30.

The cell culture chamber 15 includes an interior 35 for receiving and growing target cells in a cell culture media disposed therein, a first end 40 and a second end 45. As used herein, "target cells" refers to cells disposed within the chamber 15 and which are grown within the chamber 15. While the present disclosure is given the context of growing target cells, it will be appreciated that the system may be employed to mix chemicals or any other suitable solution or material. Also, while the first end 40 and second end 45 are shown as being at the top and bottom of the chamber 15 respectively, it will be appreciated that the ends 40 and 45 may be in any suitable orientation relative to one another (e.g., in a horizontal plane) and remain within the scope of the present disclosure. As will be discussed below, the chamber 15 may include one or more interior compartments. In addition, as will be appreciated by those skilled in the art, the chamber 15 may be formed from any suitable material, including a rigid material, a flexible material, a combination of rigid and flexible materials, a gas permeable material or any other suitable material. The chamber 15 may further include one or more ports for providing fluid communication between the chamber interior 35 and one or more reservoirs. Illustrative reservoirs include, without limitation, a cell culture media reservoir, a waste reservoir, a buffer reservoir, a CO₂ reservoir, or any other suitable reservoir.

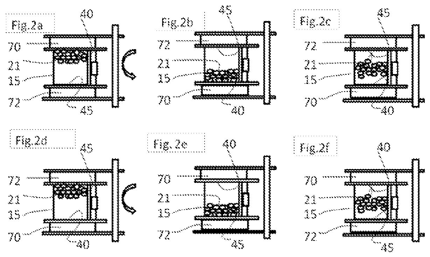

Referring now to FIG. 2a-2f, operation of the system 10 is illustrated by way of a non-limiting example. Target cells and cell culture media are delivered to the interior 35 of the chamber 15. In this embodiment, the beads 21 are buoyant and float near the top of the cell culture media within the chamber 15. In FIG. 2a, the first magnetic field generator 70 is energized and the beads 21 are held near the first end 40 of the chamber 15. The chamber 15 is then rotated approximately 180° to a position as shown in FIG. 2b wherein the first magnetic field generator 70 maintains the beads 21 near the chamber first end 40. Normally, cells are in suspension and gradually falling down to the surface of the beads 21 and the interspaces among the beads21 to start static culture. After suitable incubation period based on the culture protocol, the first magnetic field generator 70 may be de-energized whereby the beads 21 begin to float towards the second end 45 of the chamber 15 to re-suspend the cells as shown in FIG. 2c. In embodiments where the beads 21 include a coating which target cells will adhere to, movement of the beads 21 from one end to the other will collect newly grown target cells. The target cells may adhere to the beads 21 while waste is flushed from the chamber 15 and/or when new media is introduced to the chamber 15 such that a substantial number of the target cells, original and newly grown, remain within the chamber. Alternatively, magnetizable antibodies specific to the target cells may be added to the interior of chamber 15 whereby the antibodies will bind themselves to the target cells, and when a magnetic field is introduced to the chamber, the antibody bound target cells will be releasably coupled to the magnetizable beads 21 and/or the chamber wall(s) adjacent to the magnetic field generator(s). In this embodiment, one or both of the magnetic field generators 70, 72 may remain energized while unbound cells and/or waste are flushed from the chamber and/or while new media is introduced to the chamber such that a substantial number of the target cells, original and newly grown, remain within the chamber 15. Alternatively, magnetic reagents, such as Annexin V or other suitable reagent, may be employed to couple to damaged or dead cells to the beads and the healthy target cells flushed from the system 10. Further, it will be appreciated that magnetizable antibodies and/or reagents may be employed in a chamber 15 without the use of an agitator whereby the target cells or damaged/dead cells may be held against the chamber when the chamber is flushed.

Referring again to FIGS., once the beads 21 are near the second end 45 of the chamber, the second magnetic field generator 72 may be energized whereby the beads 21 are held near the chamber second end 45 (FIG 2d) and the chamber inverted to the position shown in FIG. 2e. The second magnetic field generator 72 may then be de-energized whereby the beads 21 will float towards the chamber first end 40 as shown in FIG. 2f. As will be appreciated by those skilled in the art, a variety of additives, media, buffers, CO₂ and the like may be selectively added to the chamber at any desired point during this process and/or waste selectively removed in order to promote or enhance new cell growth based on measurements taken by the control system as previously discussed. In this embodiments, the movement of beads 21 keeps the cells in suspension (dynamic) state, and when the beads 21 stop moving and keeps stay somewhere in the cell culture container (chamber), such as on the bottom of the chamber, cells slowly fall down and are evenly distributed on the surface of the beads 21 and the voids among the beads 21, from where the static cell culture starts.

In an alternative embodiment, non-buoyant beads may be employed such that the beads are moved within the chamber by rotation of the chamber and without also being subjected to magnetic fields. Here, gravity and centrifugal force, by way of rotation of the chamber, are employed to move the beads between two or more points within the chamber 15. In yet another alternative, the first and second magnetic field generators 70 and 72 may be alternately energized so as to move the beads between two or more points within the chamber and without any rotation of the chamber 15. While the forgoing example employs beads 21 as the agitator, it will be appreciated that suitable device may be employed as the agitator and remain within the scope of the present disclosure. Moreover, it will be appreciated that any means or technique for moving the agitator within the chamber may be employed and remain within the scope of the present disclosure.

Moreover, it will be appreciated that if the chamber 15 formed from gas permeable material or otherwise includes a gas permeable portion, the system may be disposed within a CO₂ incubator or CO₂ room. Without a CO₂ incubator or CO₂ room or without any gas permeable portion of the chamber, reagents, such as HEPES may be employed or, alternatively, CO₂ may be injected directly into the chamber from a CO₂ reservoir.

It will be appreciated that any feature that is described in a connection to any one embodiment may also be applicable to any other embodiment.

The agitator 20 is disposed within the chamber interior 35 and is capable of moving between the chamberfirst end 40 and chamber second end 45. Alternatively, the agitator 20 may be configured to be moved between any two or more points, or between any two or more portions, within the chamber interior 35. In the illustrative embodiment, the agitator comprises a plurality of beads 21. It will be appreciated that any illustrative embodiment showing beads 21 may use any alternative agitator configuration and remain with the scope of the present disclosure and that any particular illustrative embodiment is not limited to using beads exclusively as the agitator. In one embodiment, the beads 21 are be formed from a magnetizable material, such as silicon steel, Fe₃O₄, or any other suitable magnetizable material. As used herein, magnetizable means that the agitator, such as the beads, will hold a magnetic charge when subjected to a magnetic field but will not otherwise hold a magnetic charge once removed from the magnetic field, or the magnetic field removed from the vicinity of the agitator, for example, when a magnetic field generator is de-energized. The magnetizable material typically comprises the core of each bead 21. The magnetizable core may then be coated with any suitable material. In one embodiment, the magnetizable core is coated with polystyrene; however, it will be appreciated that the magnetizable core may be coated with any suitable material and remain within the scope of the present disclosure. For example, and without limitation, the magnetizable core may be coated with any suitable thermoplastic or thermoset polymer. While the beads 21 are shown as being formed from a magnetizable material, it will be appreciated that the beads may be formed from any suitable material, magnetizable or non-magnetizable, and remain within the scope of the present disclosure. Additionally, it will be appreciated that the beads 21 may each be coated with any suitable material such that the target cells will adhere to the beads as the cells grow within the chamber 15, yet it will be appreciated that beads not coated with a particular material to which target cells will adhere also remain within the scope of the present disclosure. In some embodiments, it may be desirable to have beads 21 that are buoyant within the cell culture media; therefore, the core of the beads may include air pockets or bubbles, a lightweight foam or plastic or any other suitable material for permitting the beads 21 to be buoyant within the media.

The beads 21 may be formed such that one or more niches, or micoenvironments, may be formed or created in the voids between the beads 21 when the beads are stacked together. In some embodiments, these niches may promote growth of additional target cells therein. In one embodiment, where the beads are substantially spherical, the diameter of each bead 21 may be between 1mm and 10mm for the creation of suitable niches. However, it will be appreciated that the beads 21 may have any suitable size and/or shape such that one or more suitable niches may be formed when the beads 21 are stacked together. Also, it will be appreciated that at least some niches may be formed between some beads and one or more walls of the chamber interior.

Referring again to FIG. 1, the control system 30 may include one or both of a controller 55 and computer 60 for controlling operation of the system 10. Alternatively, the system 10 may be run manually. The control system 30 is configured to be releasably coupled to the chamber 15. The control system 30 may include a cassette 50 for receiving the chamber 15 but it will be appreciated that the chamber 15 may be coupled to the control system 50 via a ny suitable means or configuration (e.g., clips, hooks, magnets, hook-and-loop assemblies, friction fit, etc.) and remain within the scope of the present disclosure.

The control system 30 may also include a light source 2 and a cell detector 9 for detecting the number of cells within the chamber 15, detecting the change in the number of cells within the chamber 15 or the like and reporting the results back to the control system 30. Additionally, the control system 30, via any suitable detection device, mechanism or method, may monitor the any suitable parameter involved in the growth of the target cells, for example and without limitation, the change in the number of target cells, pH, CO₂, glucose, calcium, potassium, sodium, temperature, humidity or any other suitable factor and adjust the frequency and/or speed of the movement of the agitator within the chamber and/or adjust the amount of media, the type of media, the amount of buffer, the type of buffer, the amount of CO₂, or make any other suitable adjustment based on any control system measurement so as to enhance or promote the growth of the target cells within the chamber 15.

The control system 30 is operable to cause the agitator to move within the interior 35 of the chamber 15. This may be accomplished a variety of ways. In the illustrative embodiment, the control system includes a motor 65 operable to rotate the chamber 15 between a first position and second position. As will be discussed below, the first position and second position are approximately 180° apart but it will be appreciated that first and second positions may have any suitable angular relationship relative to one another and remain within the scope of the present disclosure. The chamber 15 may be rotated in a horizontal plane, rotated in a vertical plane or rotated, shifted, slid or otherwise moved in any suitable manner to cause the agitator 20 to move within the chamber 15.

In addition, the control system 30 may include first and second magnetic field generators 70, 72 for exciting the beads 21, or other agitator 20, so as to move the beads 21 within the chamber 15 to mix the target cells and culture media. In the illustrative embodiment, each magnetic field generator is an electromagnet that generates a magnetic field when energized and ceases to create a magnetic field when de-energized. When energized, each magnetic field generator draws the agitator 20, e.g. the beads 21, toward the energized magnetic field generator. In an alternative embodiment, a permanent magnet may be used wherein the control system 30 is operable to remove the magnet from the vicinity of the chamber 15 or otherwise block the magnetic field from the magnet from penetrating into the chamber 15. While the illustrative embodiment employs both chamber rotation and electromagnets for moving the agitator within the chamber, it will be appreciated that chamber rotation may be used alone or that electromagnets may be used alone. Moreover, it will be appreciated that any technique for moving the agitator within the chamber may be employed and remain within the scope of the present disclosure.

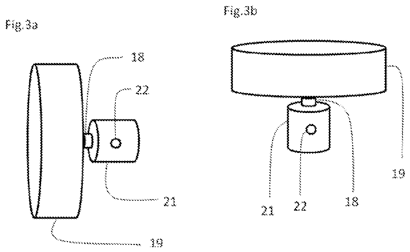

Referring now to FIG. 3, another embodiment of bioreactor for growing cells is shown. It includes a cell culture chamber (container) 19, a chamber rotation motor 21, a connector (axis) 18 between the cell culture chamber 19 and the chamber rotation motor 21, an axis 22 for the chamber position changes between vertical and horizontal. The entire bioreactor system also includes the remaining parts 2, 9, 8, 30, 55, 60 and 65 as shown in FIG. 1.

When the chamber 19 is in the vertical position, the chamber rotation motor 21 drives the chamber rotating in a certain speed (normally at 2-60 rpm) through connector 18 to keep cells in suspension state (FIG. 3a). When dynamic culture needs to stop, the motor (could be a servo 65 as shown in FIG.1.) drives the chamber to change its position from vertical to horizontal through the axis 22. During the chamber position changes, its rotating speed gradually reduces to 0 rpm to minimize the inertial effects of the cells' moving on the cell distribution. Thus, when the chamber reaches the horizontal level (FIG. 3b), cells in suspension can freely fall down to the flat bottom of the chamber 19. Although the cell chamber in FIG. 3b faces up for the static culture, but other orientation, such as faces dawn, still remains within the scope of the present disclosure. Although it is appreciated to apply the ranges for the optimal speed of the rotation (2 rpm to 60 rpm), the range for the ratio of the rotation speed /deceleration of the cell culture container rotation (1 to 160), and the range for the speed of container position change (0.1 to 60 rpm) for keep cells evenly distributed in static culture, all suitable speed of the rotation, ratio of the rotation speed /deceleration of the cell culture container rotation and speed of container position change for the cell even distribution in static culture following a dynamic culture may be used. Although the agitators, cell carriers and other supporting materials are not shown in FIG, 3a and 3b, the application of these materials in this bioreactor system may be used.

## Claims

1. A bioreactor system for cultivating cells in all three possible states: static, dynamic, or alternating between static and dynamic states, in which no cell-transferring between cell culture containers is needed during the alternating between the two culture states and all types of suspension cells, adherent cells and partial adherent cells are evenly distributed in the static cell culture following a dynamic culture, comprising
a cell culture chamber as a cell culture container for cell growth;
a power set configured to rotate the cell culture container, wherein the range for the deceleration of the cell culture container is -5 rpm² to - 120 rpm²; and
a power set configured to adjust the position of the culture container from vertical during the dynamic state and horizontal during the static state.

2. The bioreactor system according to claim 1, wherein the alternation between the two cell culture states includes from the static state to the dynamic state and from the dynamic state to the static state in which cells can be evenly distributed on the bottom of the cell culture container and/or on any materials on the bottom of the cell culture container, wherein the range for the ratio of rotation speed/deceleration of the cell culture container rotation is 1 to 160.

3. The bioreactor system according to claim 1, said cells cultivated by the bioreactor system include all types of suspension cells, adherent cells and partially adherent cells, in which the adherent cells can be attached to a certain carriers.

4. The bioreactor system according to claim 1, wherein the dynamic state includes conditions in which cells move within the medium, the medium moves around the cells, the culture container moves, or any combination thereof.

5. The bioreactor system according to claim 1, wherein the static state includes any conditions in which cells do not move relative to the cell culture container, cell culture medium, the bioreactor or the environment in which the cell culture is being performed.

6. The bioreactor systems of claim 1 can freely alternate between the dynamic state of claim 4 and the static state of claim 5, in which all alternations can occur at any time, speed, and frequency.

7. The bioreactor system in claim 1 includes a system which comprises a combination of a cell culture container inversion and magnet agitation.

8. The bioreactor system in claim 1 includes a system comprising one cell culture container orientation for rotating culture and another culture container orientation mainly for static culture.

9. The bioreactor system according to claim 1, wherein the three states, which are static, dynamic, and alternating between static and dynamic states, are programmable for and controlled by a computing system or other automation system, or are operated manually.

10. The bioreactor system according to claim 6, wherein time, speed, and frequency of the alternations between bioreactor states are programmed and controlled by a computing system, other automation system or manually.

11. The bioreactor system according to claim 1, wherein said bioreactor system includes means for preparing, incubating and harvesting the cells, as well as programming the static culture, the dynamic culture and the alternation between the static culture and the dynamic culture.

12. The bioreactor system according to claim 1, wherein said bioreactor system includes means of keeping cells evenly distributed in the static culture following a dynamic culture.

13. The bioreactor system according to claim 1, which includes a light source and a cell detector for detecting the cell number.

14. The bioreactor system according to claim 1, including means for adjusting the rotation speed in a range between 2 rpm to 60 rpm, the cell culture container deceleration in a range between -5 rpm² to -120 rpm², the ratio of rotation speed/deceleration of cell culture container rotation in a range between 1 to 160, and the speed of the container position change in a range between 0.1 rpm to 60 rpm.

## Patentansprüche

1. Bioreaktorsystem für die Kultivierung von Zellen in allen drei möglichen Zuständen: statisch, dynamisch oder zwischen statisch und dynamischen Zuständen alternierend, in welchen kein Zelltransfer zwischen Zellkulturbehältern während des Alternierens zwischen den zwei Kulturzuständen notwendig ist, und alle Arten von Suspensionszellen, anhaftenden Zellen und teilweise anhaftenden Zellen gleichmäßig in der statischen Zellkultur verteilt sind, die einer dynamischen Kultur folgt, umfassend
eine Zellkulturkammer als ein Zellkulturbehälter für das Zellwachstum;
ein Antriebsaggregat, das aufgebaut ist, um den Zellkulturbehälter zu drehen, wobei der Bereich für die Bremsung des Zellkulturbehälters -5 U/min² bis 120 U/min² ist; und
ein Antriebsaggregat, das aufgebaut ist, um die Position des Zellkulturbehälters von vertikal während des dynamischen Zustands und horizontal während des statischen Zustands einzustellen.

2. Bioreaktorsystem nach Anspruch 1, wobei das Alternieren zwischen den zwei Zellkulturzuständen von dem statischen Zustand zu dem dynamischen Zustand und von dem dynamischen Zustand zu dem statischen Zustand, in welchem Zellen gleichmäßig auf dem Boden des Zellkulturbehälters und/oder auf jedem Material auf dem Boden des Zellkulturbehälters gleichmäßig verteilt sein können, beinhaltet, wobei der Bereich des Verhältnisses der Drehgeschwindigkeit/Bremsung der Zellkulturbehälterumdrehung 1 bis 160 ist.

3. Bioreaktorsystem nach Anspruch 1, wobei die durch das Bioreaktorsystem kultivierten Zellen alle Sorten an Suspensionszellen, angehafteten Zellen und teilweise angehafteten Zellen beinhalten, in welchem die angehafteten Zellen an bestimmte Träger angehaftet sein können.

4. Bioreaktorsystem nach Anspruch 1, wobei der dynamische Zustand Bedingungen beinhaltet, in welchem die Zellen innerhalb des Mediums sich bewegen, das Medium sich um die Zellen bewegt, der Zellkulturcontainer sich bewegt oder jede Kombination davon.

5. Bioreaktorsystem nach Anspruch 1, wobei der statische Zustand alle Bedingungen beinhaltet, in welchem Zellen sich nicht relativ zu dem Zellkulturbehälter, dem Zellkulturmedium, dem Bioreaktor oder der Umgebung, in welchem die Zellkultur durchgeführt wird, bewegen.

6. Bioreaktorsystem nach Anspruch 1, das frei zwischen dem dynamischen Zustand des Anspruchs 4 und dem statischen Zustand des Anspruchs 5 alternieren kann, in welchem jedes Alternieren zu jeder Zeit, Geschwindigkeit und Frequenz auftreten kann.

7. Bioreaktorsystem in Anspruch 1, das ein System beinhaltet, welches eine Kombination einer Zellbehälterumkehrung und einer magnetischen Agitation umfasst.

8. Bioreaktorsystem in Anspruch 1, das ein System beinhaltet, das eine Zellkulturbehälterorientierung für die rotierende Kultur und eine andere Kulturbehälterorientierung hauptsächlich für statische Kultur umfasst.

9. Bioreaktorsystem nach Anspruch 1, wobei die drei Zustände, welche statisch, dynamisch und alternierend zwischen statischen und dynamischen Zuständen sind, durch ein Computersystem oder ein anderes automatisches System programmiert und gesteuert sind, oder manuell betrieben werden.

10. Bioreaktorsystem nach Anspruch 6, wobei Zeit, Geschwindigkeit und Frequenz des Alternierens zwischen Bioreaktorzuständen durch ein Computersystem, ein anderes automatisches System oder manuell programmiert und gesteuert sind.

11. Bioreaktorsystem nach Anspruch 1, wobei das Bioreaktorsystem Einrichtungen für die Zubereitung, Inkubation und Ernte der Zellen als auch für die Programmierung der statischen Kultur, der dynamischen Kultur und des Alternierens zwischen der statischen Kultur und der dynamischen Kultur beinhaltet.

12. Bioreaktorsystem nach Anspruch 1, wobei das Bioreaktorsystem Einrichtungen für die Beibehaltung der gleichmäßigen Verteilung der Zellen in der statischen Kultur, die einer dynamischen Kultur folgt, beinhaltet.

13. Bioreaktorsystem nach Anspruch 1, welches eine Lichtquelle und einen Zelldetektor für die Detektion der Zellzahl beinhaltet.

14. Bioreaktorsystem nach Anspruch 1, das Einrichtungen für die Einstellung der Umdrehungsgeschwindigkeit in einem Bereich zwischen 2 U/min bis 60 U/min, der Zellkulturbehälterabbremsung in einem Bereich zwischen -5 U/min² bis -120 U/min², des Verhältnisses der Umdrehungsgeschwindigkeit/Abbremsung der Zellkulturbehälterumdrehungen in einem Bereich zwischen 1 bis 160 und der Geschwindigkeit der Behälterpositionsänderung in einem Bereich zwischen 0,1 U/min bis 60 U/min beinhaltet.

## Revendications

1. Système de bioréacteur pour cultiver des cellules dans tous les trois états possibles : statique, dynamique, ou alterné entre les états statique et dynamique, dans lequel aucun transfert de cellules entre des récipients de culture cellulaire n'est nécessaire durant l'alternance entre les deux états de culture, et tous les types de cellules en suspension, de cellules adhérentes et de cellules partiellement adhérentes sont uniformément distribués dans la culture cellulaire statique après une culture dynamique, comprenant
une chambre de culture cellulaire en tant que récipient de culture cellulaire pour la croissance de cellules ;
un groupe d'alimentation configuré pour faire tourner le récipient de culture cellulaire, la plage pour la décélération du récipient de culture cellulaire étant de -5 t/min² à -120 t/min² ; et
un groupe d'alimentation configuré pour ajuster la position du récipient de culture à partir de la verticale durant l'état dynamique et de l'horizontale durant l'état statique.

2. Système de bioréacteur selon la revendication 1, dans lequel l'alternance entre les deux états de culture cellulaire comprend l'alternance de l'état statique à l'état dynamique et de l'état dynamique à l'état statique, dans lequel les cellules peuvent être distribuées uniformément au fond du récipient de culture cellulaire et/ou sur n'importe quels matériaux au fond du récipient de culture cellulaire, dans lequel la plage pour le rapport vitesse de rotation/décélération de la rotation du récipient de culture cellulaire est de 1 à 160.

3. Système de bioréacteur selon la revendication 1, dans lequel lesdites cellules cultivées par le système de bioréacteur comprennent tous les types de cellules en suspension, de cellules adhérentes et de cellules partiellement adhérentes, dans lequel les cellules adhérentes peuvent être attachées à certains supports.

4. Système de bioréacteur selon la revendication 1, dans lequel l'état dynamique comprend des conditions dans lesquelles les cellules se déplacent dans le milieu, le milieu se déplace autour des cellules, le récipient de culture bouge, ou l'une quelconque de leurs combinaisons.

5. Système de bioréacteur selon la revendication 1, dans lequel l'état statique comprend n'importe quelles conditions dans lesquelles les cellules ne se déplacent pas par rapport au récipient de culture cellulaire, au milieu de culture cellulaire, au bioréacteur ou à l'environnement dans lequel la culture cellulaire est effectuée.

6. Système de bioréacteur selon la revendication 1, qui peut alterner librement entre l'état dynamique de la revendication 4 et l'état statique de la revendication 5, dans lequel toutes les alternances peuvent se produire à n'importe quels moment, vitesse et fréquence.

7. Système de bioréacteur selon la revendication 1, qui comprend un système comprenant une combinaison d'une inversion de récipient de culture cellulaire et d'une agitation magnétique.

8. Système de bioréacteur selon la revendication 1, qui comprend un système comprenant une orientation du récipient de culture cellulaire pour la culture en rotation et une autre orientation principalement pour la culture statique.

9. Système de bioréacteur selon la revendication 1, dans lequel les trois états, qui sont statique, dynamique, et alterné entre les états statique et dynamique, sont programmable pour et commandés par un système informatique ou un autre système d'automatisation, ou sont opérés manuellement.

10. Système de bioréacteur selon la revendication 6, dans lequel le temps, la vitesse et la fréquence des alternances entre les états du bioréacteur sont programmés et commandés par un système informatique, par un autre système d'automatisation, ou manuellement.

11. Système de bioréacteur selon la revendication 1, lequel système de bioréacteur comprend des moyens pour préparer, incuber et récolter les cellules, ainsi que pour programmer la culture statique, la culture dynamique et l'alternance entre la culture statique et la culture dynamique.

12. Système de bioréacteur selon la revendication 1, lequel système de bioréacteur comprend des moyens pour maintenir les cellules uniformément distribuées dans la culture statique après une culture dynamique.

13. Système de bioréacteur selon la revendication 1, qui comprend une source de lumière et un détecteur de cellules pour détecter le nombre de cellules.

14. Système de bioréacteur selon la revendication 1, qui comprend des moyens pour ajuster la vitesse de rotation dans la plage comprise entre 2 t/min et 60 t/min, la décélération du récipient de culture cellulaire dans la plage comprise entre - 5 t/min² et -120 t/min², le rapport vitesse de rotation/décélération de la rotation du récipient de culture cellulaire dans la plage comprise entre 1 et 160 et la vitesse du changement de position du récipient dans la plage comprise entre 0,1 t/min et 60 t/min.
